(19) Europäisches Patentamt / European Patent Office / Office européen des brevets

(11) **EP 3 548 124 B1**

(12) **EUROPEAN PATENT SPECIFICATION**

(45) Date of publication and mention
of the grant of the patent:
**24.06.2026 Bulletin 2026/26**

(21) Application number: **17817513.9**

(22) Date of filing: **29.11.2017**

(51) International Patent Classification (IPC):
**A61M 5/44** *(2006.01)*    **A61M 5/36** *(2006.01)*
**A61M 5/168** *(2006.01)*    **G05D 7/00** *(2006.01)*

(52) Cooperative Patent Classification (CPC):
**A61M 5/44; A61M 5/16804; A61M 5/365;**
**G05D 23/19; H05B 6/06; H05B 6/108; H05B 6/109;**
A61M 5/16886; A61M 2205/127; A61M 2205/3331

(86) International application number:
**PCT/US2017/063612**

(87) International publication number:
**WO 2018/102354 (07.06.2018 Gazette 2018/23)**

(54) **RAPID INFUSER WITH ADVANTAGEOUS FLOW PATH FOR BLOOD AND FLUID WARMING**

SCHNELLINFUSIONSVORRICHTUNG MIT VORTEILHAFTEM STRÖMUNGSWEG ZUR
ERWÄRMUNG VON BLUT UND FLÜSSIGKEIT

DISPOSITIF DE PERFUSION RAPIDE DOTÉ D'UN TRAJET D'ÉCOULEMENT AVANTAGEUX
PERMETTANT DE RÉCHAUFFER DU SANG ET DU FLUIDE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **30.11.2016   US 201615364499**
**30.11.2016   US 201615364515**
**30.11.2016   US 201615364532**

(43) Date of publication of application:
**09.10.2019 Bulletin 2019/41**

(73) Proprietor: **Belmont Instrument, LLC**
**Billerica, Massachusetts 01821 (US)**

(72) Inventors:
• **LANDY, III, John J.**
**Billerica, Massachusetts 01821 (US)**

• **BRUSARD, George G.**
**deceased (US)**
• **DION, Tristan**
**Billerica, Massachusetts 01862 (US)**
• **TSENG, Yeu Wen**
**Watertown, Massachusetts 02472 (US)**

(74) Representative: **Graham Watt & Co**
**St. Botolph's House**
**7-9 St. Botolph's Road**
**Sevenoaks TN13 3AJ (GB)**

(56) References cited:
CN-Y- 201 012 173        GB-A- 1 208 925
US-A- 4 694 976          US-A- 5 319 170
US-A1- 2001 025 160      US-A1- 2009 012 655
US-A1- 2014 309 612      US-B1- 6 280 422

**Description**

BACKGROUND

To treat hemorrhaging (e.g., escape of blood from a ruptured vessel), it is often necessary to quickly infuse a substantial volume of fluid, e.g. whole blood, plasma or blood substitute, so as to maintain an adequate blood volume and circulation. However, to preserve blood or blood products, such materials are typically refrigerated, and it is necessary to warm them before infusion so as to avoid shock to a patient's system. The fluid or infusate (e.g., blood or blood product) is typically warmed using a fluid warmer. While it is desired to heat the infusate quickly, the temperature of the infusate should not exceed 40 °C to 42 °C, since at higher temperatures, protein denaturation and red cell damage can occur. It is also important that any electrical power or potentials utilized in the heating process be effectively isolated from a patient.

Many rapid fluid warmers utilize a relatively large water bath reservoir which is preheated to 39 °C to 40 °C. Water is pumped rapidly through the heat exchanger through which an infusate (blood or other infusate) is perfused, the two fluids being separated by a thin, usually metallic, heat exchange surface. These devices are relatively large and cumbersome due to the need for a water bath and water pump, as well as the heat exchanger and associated conduits. Additionally, there may be a risk of contamination of infusate with heat exchanger water.

Some dual flow path fluid warmers (e.g., the Belmont® Rapid Infuser, the Belmont® Hyperthermia Pump) utilize inductive or electro-magnetic heating techniques, involving a circular heat exchanger tailored to the shape of the magnetic field. The electro-magnetic heater includes a primary inductor (coil), generating an alternating magnetic field. A high current density in the secondary inductors (e.g., multiple thin ribbon like conductors placed in parallel) converts electric energy into thermal energy, so that the secondary inductors provide heat to a fluid that is in direct contact with the secondary inductors.

Fluid warmers may be utilized to supply fluids or infusates (e.g., blood, blood products) at relatively high flow rates (e.g., greater than 500 ml/min, or greater than 750 ml/min) particularly during medical emergencies. Administration of cold blood and other fluids at high flow rates using an in line warming system requires the delivery of high quantities of power to the fluids (e.g., to heat the fluid). To infuse cold fluids at a rate in excess of these levels would require more energy than the typical AC outlet can supply. To overcome this issue, certain systems store thermal energy in a separate fluid (e.g., water or oil, not infusate) for transferring the stored heat from the fluid to the infusate during times of need. Drawbacks of this approach include the necessity to later transfer energy to the infusate before/during infusion with the limited heat transfer rate of the system (e.g., due to a limited surface area between water/oil and infusate) and the potential contamination of the infusate by the fluid bath. Thus, better systems and methods to store and transfer thermal energy at high flow rates that minimize energy utilization and heat times are required.

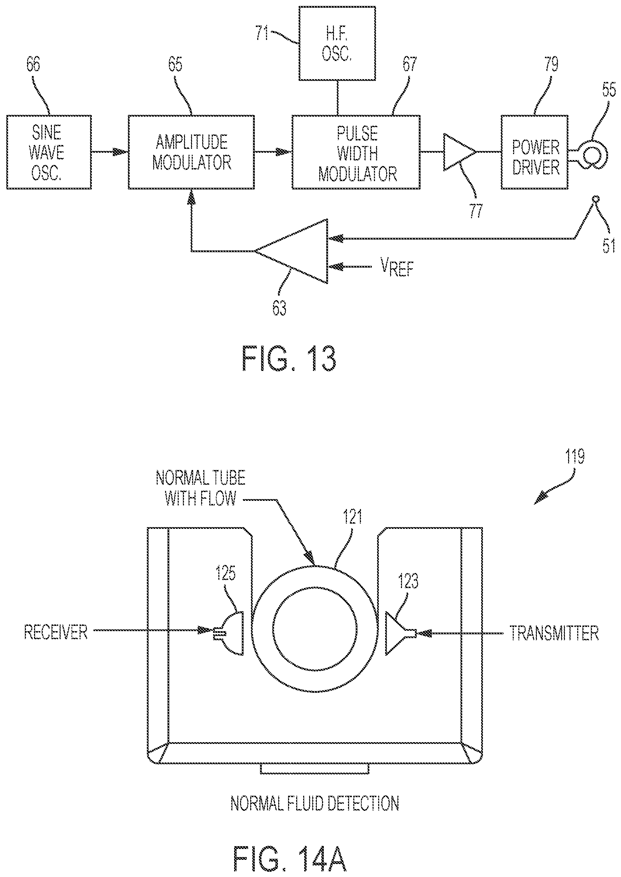

Fluid warmers often have a sensor connected to inflow tubing to detect if an infusate reservoir is empty. Typically, the sensor comprises a transmitter and a receiver (FIG. 14A-14C), and measures velocity of ultrasound waves from the transmitter to distinguish between fluid and air, since ultrasound waves travel faster through fluids than air. The inflow tubing is located between the transmitter and the receiver. When the fluid level in the infusate reservoir is low (or zero), the pressure in the infusate reservoir and the inflow tubing can be lower than the atmospheric pressure. Therefore, the non-rigid inflow tubing deforms to minimize the pressure difference. The fluid out sensor measures this deformation in inflow tubing to detect fluid availability in the tubing and therefore, the reservoir.

The direction of deformation affects detectability of fluid levels in the inflow tubing by the sensor. The pressure difference between the inside of the non-rigid inflow tubing and the outside atmospheric pressure may result in the tubing collapsing from a circular cross-section into an elliptical cross-section. For example, the major axis of the elliptical cross-section of the collapsed inflow tubing may be perpendicular to a line between the transmitter and the receiver, or may be parallel to a line between the transmitter and the receiver. In some instances, e.g., where the major axis of the elliptical cross-section of the collapsed inflow tubing is perpendicular to a line between the transmitter and the receiver of the fluid out sensor, the sensor can detect the deformation due to the presence of air in the ultrasound beam path. However, in some instances, e.g., where the major axis of the elliptical cross-section of the collapsed inflow tubing is parallel to a line between the transmitter and the receiver, the sensor cannot detect the deformation due to lack of air in the ultrasound beam path. Improvements to existing sensors or incorporation of additional components that prevent the undesired orientation of a deformed inflow tubing are necessary for efficient and accurate functioning of the fluid heating system.

Components of fluid heating systems (e.g., heat exchangers, sensors, thermal energy storage) as described in U.S. Patent Nos. 5,319,170, 7,819,835, and 9,737,672, can be utilized in hyperthermia pump systems, and rapid fluid infusers.

Hyperthermia refers to the increase in an individual's body temperature above his or her normal range. Research has shown that temperatures greater than 41 °C can damage cancer cells, usually without significant damage of normal tissues. While there are multiple methods of inducing hyperthermia by either direct skin contact or radiant heating, many physicians favor an extracorporeal heat exchange (blood) circuit to raise patient body temperatures. Temperature and time may be interrelated with respect to tumor necrosis and risk of toxicity to normal cells. The inefficiency of tumor cells to dissipate heat due to their disorganized and compact vascular structure subjects the tumor to hypoxia, anaerobic metabolism, and local acidosis, making the tumor more vulnerable to injury and causing the tumor cells to undergo apoptosis.

Fluid heating systems are used regularly for various other kinds of medical treatments, in addition to hyperthermia treatment (e.g., treatment of hemorrhaging, treatment of tumors, treatment of circulating tumor cells (CTCs)). Further improvements to existing heating system technologies that ensure higher efficiencies and lower heating times at a wide range of flow rates are needed. In the prior art, US 2009/012655 A1 relates to dialysis fluid heating algorithms. US 5319170 A relates to an induction fluid heater utilizing a shorted turn linking parallel flow paths. US 6,280,422 B1 relates to feeding apparatus with replaceable feeding bottle. US 2001/025160 A1 relates to a fluid management assembly having a vented outlet line for use in endoscopic procedures.

SUMMARY

The invention provides a system for heating a fluid, according to the appended claims. The present disclosure provides improved technologies relating to medical fluid heating systems and apparatus. In certain embodiments, the present disclosure relates to systems and apparatus for heating a fluid and, more particularly, for quickly and controllably heating a flow of blood or a blood product, which may be needed, for example, for infusion into a patient or for hyperthermia treatment. In particular, the present disclosure is directed to a system and apparatus featuring single flow path fluid heating ("single path flow"). Moreover, the present disclosure is directed to a system and apparatus for efficiently utilizing the thermal energy of an infusate stored in a reservoir ("slack time heating"). Furthermore, the present disclosure is directed to a fluid heating system and apparatus featuring a vacuum release valve designed to prevent the undesired orientation of deformed inflow tubing ("vacuum release valve").

With respect to the "single flow path" technology, a new fluid flow design is described herein that improves on prior strategies for heating infusate (e.g., blood, hyperthermia fluid). In particular, the design addresses an occluding issue that may result in overheating of the fluid and other inefficiencies. In certain embodiments, the design offers an improvement on inductive or electro-magnetic heating systems with toroidal dual flow paths.

For example, at low flow rates, the pressure drop from dual flow paths to the outlet may be insufficient to support flow through both paths, causing flow to occur only on a single side of the dual flow path. The other side of the dual flow path, therefore, may encounter stagnant or slow flow. The stagnant or slow flow in a toroidal heat exchanger may become an issue if, for example, blood or blood products have either been improperly anticoagulated or have had the anticoagulant compromised (e.g., mixed with lactated Ringer's or other solutions containing calcium). If the improperly treated blood or blood products coagulate in one of the flow paths with stagnant or slow flow, that flow path may become clogged. When the system with the occluded heat exchanger starts to supply heated fluids at a sufficiently high rate through the non-clogged path (e.g., with an increased heat supply), the heat exchanger may overheat, leading to system damage.

Embodiments of the present disclosure include new designs that provide a single flow path while retaining the advantages of certain existing blood warmers with inductive or electro-magnetic heaters. The newly designed single flow path heat exchanger would not experience stagnant flow even under conditions of improperly treated fluids or compromised anticoagulants at low rates that, in previous designs, may have caused overheating of a local flow path. The single flow path is found to obviate problems that may occur with the dual flow path system due to improper usage (e.g., use of blood or blood products that have not been properly treated or anticoagulated). In certain embodiments, the system retains advantages of toroidal electromagnetic heat exchangers, such as the ability to provide a wide range of accurately controlled flow rates, for example, flow rates that span a range from 2.5 mL/min to 1000 mL/min, and any range in between.

With respect to the "slack time heating" technology, embodiments described herein address a problem that arises with infusate heating systems that store thermal energy in a separate fluid (e.g., water, not infusate) for heat transfer to an infusate. Such systems, as previously noted, typically utilize a relatively large preheated water bath reservoir to transfer heat from the water to the infusate. Drawbacks of this approach include a relatively low heat exchange efficiency due to the limited heat exchange surface area between the water and the infusate. Another drawback is the potential contamination of the infusate by the water bath. Thus, "slack time heating" systems and methods are presented herein for efficiently utilizing the thermal energy of an infusate stored in a reservoir. In certain embodiments, an infusate is heated by an induction heater e.g., an electromagnetic heater, and thermal energy of thusly heated infusate (e.g., not water or other non-infusate heat exchange fluid) is stored in a reservoir. When the heater is not being actively used to infuse a patient, infusate is sent through the induction heater, is warmed, and is sent to a reservoir. Then, when the infusate is sent to the patient, fluid from the reservoir again passes through the induction heater before being sent to the patient. The amount of thermal energy that needs to be transferred to the fluid (e.g., infusate) is smaller, if the temperature difference the heater needs to achieve to heat the fluid (e.g., infusate) is lower (e.g., if the temperature of fluid going into the heater from the reservoir is higher). Thus, during a medical procedure, when the infuser is not needed, fluid can pass through the heater, and then reside in the reservoir until needed, thereby storing thermal energy. Thus, the system can heat the infusate more rapidly when the infusion is needed. As the system does not use other fluids to heat the infusate, the system also does not suffer from a risk of contamination, unlike water-based heat exchangers.

With respect to the "vacuum release valve" technology, embodiments of the present disclosure are directed to a fluid heating system that includes a vacuum release valve to prevent the undesired orientation of the deformed inflow tubing that occurs when the fluid levels in the tubing and/or reservoir are low or zero. The vacuum release valve supplies air to the tubing to reduce pressure difference between the inflow tubing and the surroundings, preventing the deformation of the inflow tubing.

In one aspect, the invention is directed to a system for heating a fluid, which comprises a conduit (e.g., toroid-shaped) defining a central opening, a primary inductor at least partially within the central opening, one or more secondary inductors within the conduit (e.g., wherein each of the one or more secondary inductors is ring-shaped, flat), the conduit defining a central opening, an inflow tubing connected to the conduit, and a vacuum release valve. In the invention, each of the one or more secondary inductors is substantially parallel. In the invention, the secondary inductors are separated by gaps (or spaces). In the invention, the primary inductor comprises a coil (or a winding). In the invention, when the primary inductor is energized, the coil generates magnetic flux passing through the central opening. In the invention, when pressure in the inflow tubing is lower than atmospheric pressure, the vacuum release valve allows flow of air into the inflow tubing, thereby rapidly allowing the detection of a fluid out condition.

In the invention, the system further comprises a reservoir for containing an infusate. In the invention, the vacuum release valve is connected to the reservoir. In certain embodiments, the vacuum release valve is connected to the top of the reservoir. In certain embodiments, the vacuum release valve is connected to the bottom of the reservoir. In certain embodiments, the reservoir further comprises a tubing attached to the top of the reservoir, and the vacuum release valve is connected to the reservoir through the tubing.

In certain embodiments, the vacuum release valve allows flow of air into the inflow tubing when pressure in the inflow tubing is 1, 2, 3, 4, 5, 6, 7, 8, 9, or 10 psi lower than atmospheric pressure.

In certain embodiments, the conduit provides a single fluid flow path. In certain embodiments, the single fluid flow path has a conformation such that fluid is not in contact with the primary inductor.

In certain embodiments, the system further comprises a fluid separator comprising an inlet nozzle and an outlet nozzle, wherein the inlet nozzle directs an inlet flow to the conduit through the fluid separator and the outlet nozzle receives an outlet flow from the conduit through the fluid separator.

In certain embodiments, the conduit comprises a plurality of spacers which provides the gaps between the secondary inductors.

In certain embodiments, wherein the one or more secondary inductors transfers heat to fluid within the conduit.

In certain embodiments, the system further comprises a bubble trap for removing air bubbles from fluid flowing through the system.

BRIEF DESCRIPTION OF THE DRAWINGS

The Drawings, which are comprised of at least the following figures, are for illustration purposes only, not for limitation.

The foregoing and other objects, aspects, features, and advantages of the present disclosure may become more apparent and better understood by referring to the following description taken in conduction with the accompanying drawings, in which:

FIG. 1 is a schematic block diagram of a fluid heating apparatus, according to an illustrative embodiment of the present invention.

FIG. 2 is a front view of a disposable set, according to an embodiment of the instant invention.

FIG. 3 is a cross-sectional view of an inductive heater employed, shown with a primary inductor, secondary inductors, a conduit, an inlet and an outlet, according to an illustrative embodiment of the present invention.

FIG. 4 is a plan view, with parts broken away, of the heater of FIG. 3.

FIG. 5 illustrates a single flow path conduit with a fluid separator comprising an inlet nozzle and an outlet nozzle, according to an embodiment of the present invention.

FIGS. 6A-6G illustrate a fluid separator, according to an embodiment of the present invention.

FIGS. 7A and 7B are optical images of a fluid separator, according to an embodiment of the present invention.

FIG. 8 shows a part of a disposable set with a fluid separator, a conduit and a plurality of secondary inductors, according to an embodiment of the present invention.

FIGS. 9A-9E depict a spiral inductive tube, according to an embodiment of the present invention.

FIGS. 10A-10E depict a spiral inductive tube, according to other embodiments of the present invention.

FIG. 11 shows a spiral inductive tube, according to an embodiment of the present invention.

FIG. 12 is a schematic representation of a slack time heating system with an associated disposable set, according to an embodiment of the present invention.

FIG. 13 is a schematic block diagram of circuitry for energizing the inductive heater of FIG. 3 and FIG. 4.

FIGS. 14A-14C demonstrate a sensor for a fluid level in a reservoir, and deformation of an inflow tubing.

FIGS. 15A-15C depict exemplary disposable set with vacuum release valve, according to an embodiment of the present invention.

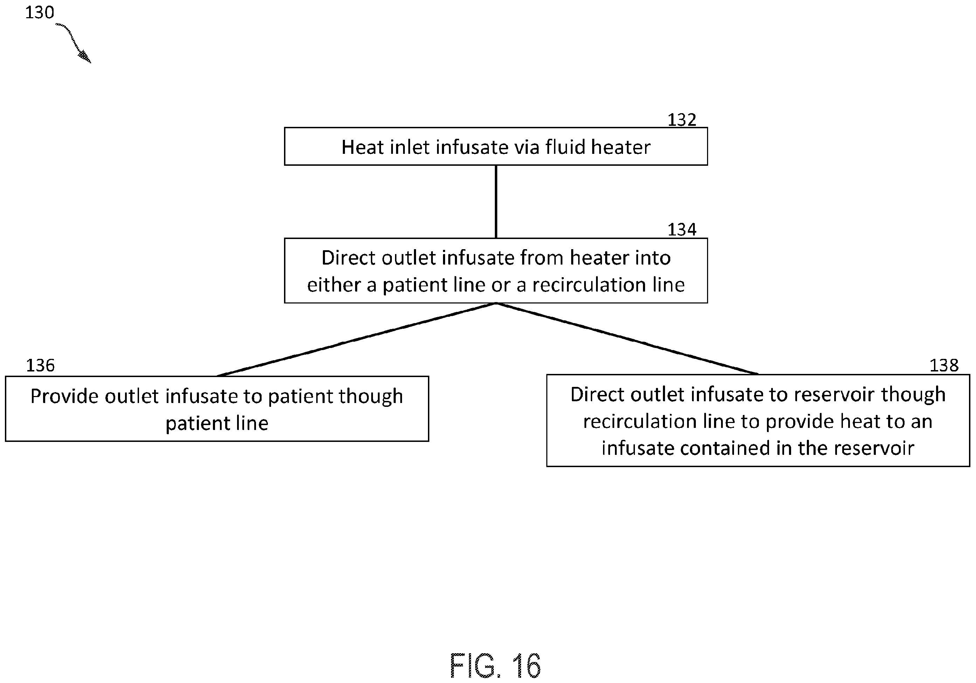

FIG. 16 depicts a method for heating an infusate.

DEFINITIONS

In order for the present disclosure to be more readily understood, certain terms are first defined below. Additional definitions for the following terms and other terms are set forth throughout the specification.

As used herein, the term "administration" typically refers to the administration of a composition to a subject or system. Those of ordinary skill in the art will be aware of a variety of routes that may, in appropriate circumstances, be utilized for administration to a subject, for example a human. For example, in certain embodiments, administration may be ocular, oral, parenteral, topical, etc. In some particular embodiments, administration may be bronchial (e.g., by bronchial instillation), buccal, dermal (which may be or comprise, for example, one or more of topical to the dermis, intradermal, interdermal, transdermal, etc.), enteral, intra-arterial, intradermal, intragastric, intramedullary, intramuscular, intranasal, intraperitoneal, intrathecal, intravenous, intraventricular, within a specific organ (e. g. intrahepatic), mucosal, nasal, oral, rectal, subcutaneous, sublingual, topical, tracheal (e.g., by intratracheal instillation), vaginal, vitreal, etc. In certain embodiments, administration may involve dosing that is intermittent (e.g., a plurality of doses separated in time) and/or periodic (e.g., individual doses separated by a common period of time) dosing. In certain embodiments, administration may involve continuous dosing (e.g., perfusion) for at least a selected period of time.

As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

The term "biocompatible", as used herein, refers to materials that do not cause significant harm to living tissue when placed in contact with such tissue, e.g., in vivo. In certain embodiments, materials are "biocompatible" if they are not toxic to cells. In certain embodiments, materials are "biocompatible" if their addition to cells in vitro results in less than or equal to 20% cell death, and/or their administration in vivo does not induce significant inflammation or other such adverse effects.

A composition or method described herein as "comprising" one or more named elements or steps is open-ended, meaning that the named elements or steps are essential, but other elements or steps may be added within the scope of the composition or method. To avoid prolixity, it is also understood that any composition or method described as "comprising" (or which "comprises") one or more named elements or steps also describes the corresponding, more limited composition or method "consisting essentially of" (or which "consists essentially of") the same named elements or steps, meaning that the composition or method includes the named essential elements or steps and may also include additional elements or steps that do not materially affect the basic and novel characteristic(s) of the composition or method. It is also understood that any composition or method described herein as "comprising" or "consisting essentially of" one or more named elements or steps also describes the corresponding, more limited, and closed-ended composition or method "consisting of" (or "consists of") the named elements or steps to the exclusion of any other unnamed element or step. In any composition or method disclosed herein, known or disclosed equivalents of any named essential element or step may be substituted for that element or step.

As used herein, the term "designed" refers to an agent (i) whose structure is or was selected by the hand of man; (ii) that is produced by a process requiring the hand of man; and/or (iii) that is distinct from natural substances and other known agents.

As used herein, the term "low flow rate" refers to a non-zero (e.g., no less than 1 ml/min) flow rate less than about 100 ml/min, or less than about 50 ml/min, or less than about 40 ml/min, or less than about 30 ml/min, or less than about 20 ml/min, or less than about 15 ml/min, or less than about 10 ml/min.

As used herein, the term "patient" refers to any organism to which a provided composition is or may be administered, e.g., for experimental, diagnostic, prophylactic, cosmetic, and/or therapeutic purposes. Typical patients include animals (e.g., mammals such as mice, rats, rabbits, non-human primates, and/or humans). In certain embodiments, a patient is a human. In certain embodiments, a patient is suffering from or susceptible to one or more disorders or conditions. In certain embodiments, a patient displays one or more symptoms of a disorder or condition. In certain embodiments, a patient has been diagnosed with one or more disorders or conditions. In certain embodiments, the disorder or condition is or includes cancer, or presence of one or more tumors. In certain embodiments, the patient is receiving or has received certain therapy to diagnose and/or to treat a disease, disorder, or condition.

As used herein, the term "substantially" refers to the qualitative condition of exhibiting total or near-total extent or degree of a characteristic or property of interest.

As used herein, the term "treatment" (also "treat" or "treating") refers to any administration of a therapy that partially or completely alleviates, ameliorates, relives, inhibits, delays onset of, reduces severity of, and/or reduces incidence of one or more symptoms, features, and/or causes of a particular disease, disorder, and/or condition. Such treatment may be of a subject who does not exhibit signs of the relevant disease, disorder, and/or condition and/or of a subject who exhibits only early signs of the disease, disorder, and/or condition. Alternatively or additionally, such treatment may be of a subject who exhibits one or more established signs of the relevant disease, disorder, and/or condition. Treatment may be of a subject who has been diagnosed as suffering from the relevant disease, disorder, and/or condition. Treatment may be of a subject known to have one or more susceptibility factors that are statistically correlated with increased risk of development of the relevant disease, disorder, and/or condition.

DETAILED DESCRIPTION

Throughout the description, where compositions are described as having, including, or comprising specific components, or where methods are described as having, including, or comprising specific steps, it is contemplated that, additionally, there are compositions of the present invention that consist essentially of, or consist of, the recited components, and that there are methods that consist essentially of, or consist of, the recited processing steps.

It should be understood that the order of steps or order for performing certain action is immaterial so long as the invention remains operable. Moreover, two or more steps or actions may be conducted simultaneously.

The following description is for illustration and exemplification of the disclosure only, and is not intended to limit the invention to the specific embodiments described.

The mention herein of any publication, for example, in the Background section, is not an admission that the publication serves as prior art with respect to any of the claims presented herein. The Background section is presented for purposes of clarity and is not meant as a description of prior art with respect to any claim. All headers and subheaders are provided for the convenience of the reader - they are not intended to limit applicability of the content that follows them.

As mentioned above, the present disclosure relates to warming of fluid (e.g., blood, blood products, hyperthermia fluid, and more). The present disclosure encompasses system, apparatus, and/or methods of warming fluids.

Most rapid fluid warmers utilize a relatively large water bath reservoir which is preheated to 39 °C to 40 °C. Water is pumped rapidly through a heat exchanger through which the infusate is perfused, the two fluids typically being separated by a thin, usually metallic, heat exchange surface. These devices are relatively large and cumbersome due to the need for the water bath and water pump, as well as a heat exchanger and associated conduits. Additionally, there may be a risk of contamination of infusate with heat exchanger water. Such devices accordingly are not ideal for use in emergency situations.

Other rapid fluid warmers utilize resistive heating (i.e., Joule heating) to increase temperature of the infusate. The resistive heaters are smaller than heaters with the water bath. However, resistive heaters are often inaccurate and can cause overheating the infusate. Typically, the infusate does not contact the resistive heater directly, receiving heat through a wall with a high thermal resistance (e.g., a wall of an infusate container made of plastic). The resistive heater maintains a high temperature difference between the heater and the infusate to compensate for the high thermal resistance of the wall or the infusate container. Therefore, a local temperature of the resistive heaters can reach up to about 80 °C, denaturing proteins in the infusate.

Commercially available rapid fluid warmers are described in, for example, US Patent Nos. 5,319,170, 6,175,688, 6,236,809, 6,480,257, and 7,819,835.

Some blood warmers (e.g., the Belmont® Rapid Infuser, the Belmont® Hyperthermia Pump) utilize inductive or electro-magnetic heating techniques, involving a circular heat exchanger tailored to the shape of the magnetic field. Fluid is introduced into the toroid at a point where the fluid path bifurcates with each half flowing on opposites sides of the toroid, until the two halves rejoin at the fluid output of the toroid. The electro-magnetic heater includes a primary inductor (coil), generating an alternating magnetic field. A high current density in the secondary inductors (e.g., multiple thin ribbon like conductors placed in parallel) converts electric energy into thermal energy, so that the secondary inductors provide heat to a fluid. The infusate is in direct contact with the secondary inductors and receives heat directly from the secondary inductors without involving a wall with a high thermal resistance. This effective heat exchange system of the Belmont® Rapid Infuser enables fluid (e.g., blood, blood products with temperature of 4 to 37.5 °C) to be heated to a target temperature (e.g., normothermia) in a single pass. The efficiency of the system is higher than other systems as heat is exchanged through direct contact between the infusate and secondary conductors without involving a wall (e.g., infusate container) with a high thermal resistance.

In these systems, a user can control heat flux to the fluid precisely, thus, the electro-magnetic heating system prevents overheating. The toroidal blood warmer is equipped with a large digital touch screen display (e.g., LCD type with water proof touch pad) that displays step-by-step instructions for easy use and control. The large touch screen continuously displays fluid temperature, total volume infused, line pressure, and both target and actual infusion rates. The blood warmer is also equipped with automatic air removal with two air detectors one at input and another at output. This ensures that no air bubbles are accidentally introduced into the patient. There are sensors and automatic alarms to avoid unsafe conditions, for example if the line becomes obstructed, when the reservoir is out of fluid or when other conditions occur. Other sensors include pressure transducer, temperature probes (e.g., infrared sensing), pump infusion rate sensor, open door detector, and valve activation sensors. The blood warmers may be powered by Alternating Current (AC) power or on battery (e.g., for mobility). Furthermore, the size of the Belmont® Rapid Infuser and the Belmont® Hyperthermia Pump is significantly smaller (e.g., IV-pole mountable) than other commercial fluid heaters and has an extra-long power cord (e.g., 15 foot) for increased mobility.

Embodiments described herein provide an improvement on the aforementioned toroidal heat exchangers with dual flow paths. For example, during operation of the system at low flow rates, the pressure drop from the flow paths to the outlet may be insufficient to support flow through both paths, causing flow to occur only on a single side of the dual flow path. The other side of the dual flow path, therefore, encounters stagnant or slow flow. Normally, this does not pose a concern; however, the stagnant or slow flow in a toroidal heat exchanger may create a potential issue when blood or blood products have either been improperly anticoagulated or have had the anticoagulant compromised (e.g., mixed with lactated Ringer's or other solution containing calcium). In such cases, clogs may occur. For example, if improperly treated blood or blood product coagulates in one of the flow paths which have stagnant or slow flow, that flow path may become clogged. When the system with the clogged flow path starts to supply heated fluids at a sufficiently high rate (e.g., with an increased heat supply to the heat exchangers), the clogged flow path can overheat due to insufficient supply of fluids.

Embodiments of the present disclosure include new designs that provide a single flow path. The newly designed single flow path heat exchanger would not experience stagnant flow even under conditions of low flow rates and improper or compromised anticoagulants that, in previous designs, may have resulted in overheating of a local flow path. The single flow path is found to obviate problems that may occur with the dual flow path system due to improper usage (e.g., use of blood or blood products that have not been properly anticoagulated).

## Fluid heating system

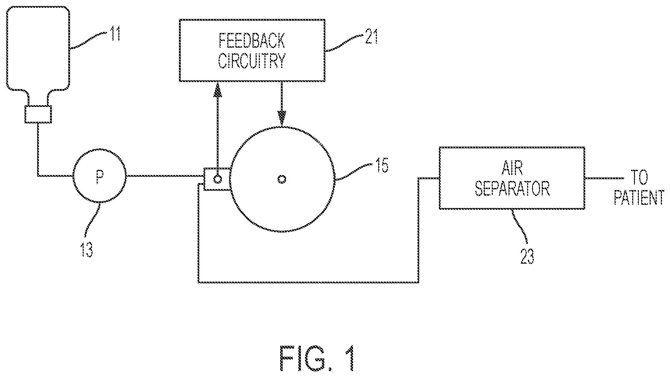

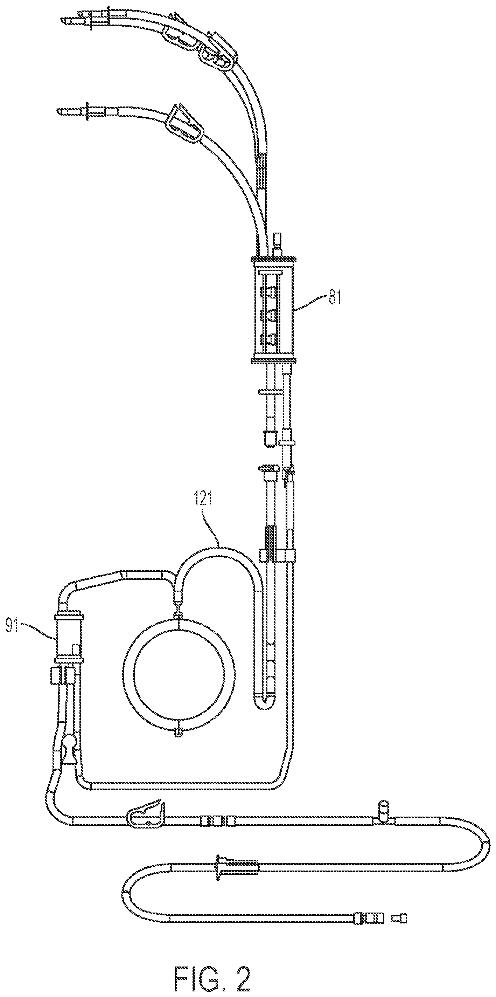

In the invention, as illustrated in FIGS. 1 and 2, a fluid heating system comprises a reservoir of infusate (e.g., blood, plasma or other solution). Fluid tubular lines and connectors of an infusion fluid disposable set, for example, as described in FIG. 2, direct fluid (e.g., infusate) to reservoirs of the infusion fluid disposable set.

Additional tubular fluid lines and connectors of a disposable set direct fluid from reservoirs to and through a heater. Infusate drawn from reservoir 11 is driven by a pump 13 (e.g., roller pump) through an inductive heater constructed in accordance with the present disclosure. Heated fluid is directed into a patient tubular fluid feed line to a patient's body.

In heater 15, infusate is brought to a preselected temperature; control of the temperature is affected by feedback circuitry 21 that responds to outlet temperature, e.g., as sensed by a temperature sensor 51 of FIG. 13 to control the energization of an inductor, which effects the heat generation in the heater 15. This feedback circuitry is described in greater detail below. From the heater 15, the infusate passes through a separator 23, which removes any possible trapped air bubbles and then passes to the patient.

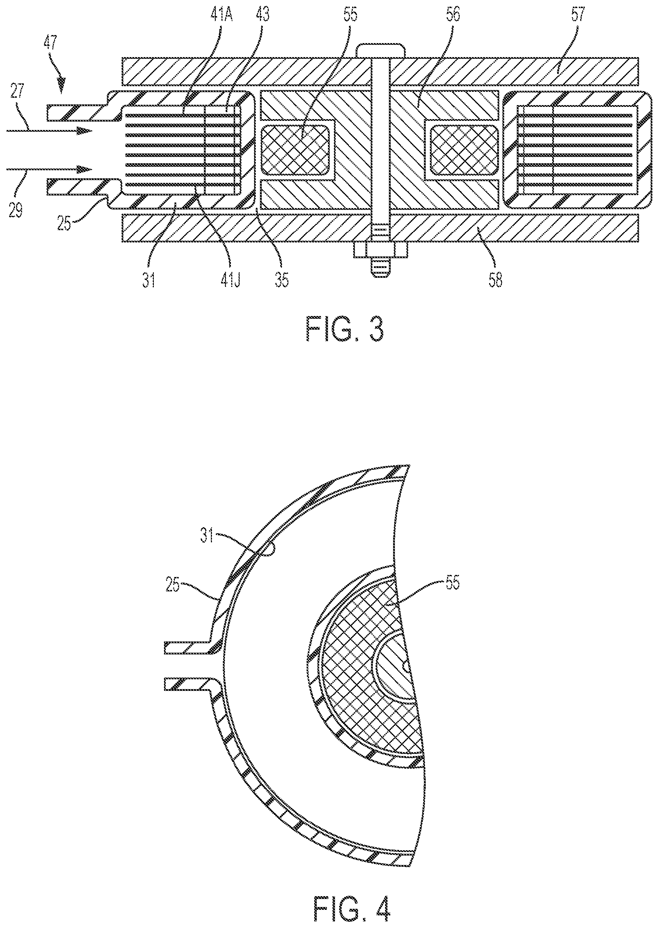

In the invention, as is illustrated in greater detail in FIGS. 3 and 4, the heater 15 involves a conduit or housing 25, which may be constructed of a suitable plastic material. A fluid separator 47 (e.g., made of the same plastic material) provides physical separation between an inlet 27 and an outlet 29, which are connected by a ring-like annular chamber 31. This defines a single circular flow path from the inlet 27 to the outlet 29 with an opening 35 in the middle. While the exemplary path is shown as forming a circle, it should be understood that other shapes could also be used, e.g. ovoid.

Within the chamber 31, there is a plurality of thin or ribbon-like secondary conductors 41A-41J. As illustrated, the conductors are in the shape of circular rings but it should be understood that other shapes could also be used, for example, ovoid.

A primary inductor comprising a winding 55 wound on a ferrite bobbin core 56 generates magnetic flux passing through the central opening and is inductively coupled to the plurality of secondary inductors 41A-41J for inducing local currents therein. The winding, however, does not surround the flow path. To improve the degree of coupling between the winding 55 and the secondary inductors 41A-41J, ferrite magnetic end plates 57 and 58 may be employed to extend the flux coverage.

## Heat exchanger

In certain embodiments, the heat exchanger comprises thin or ribbon-like secondary inductors 41A-41J contained in a chamber 31. As illustrated, the secondary inductors have a circular ring-like shape. In alternative embodiments, other shapes (e.g., beads, ovoid) can be used. In certain embodiments, the secondary inductors extend generally parallel to each other in a spaced relationship (spaced apart) with each secondary inductor passing through the flow path so as to heat the fluid. As will be apparent, fluid flowing from the inlet 27 to outlet 29 will pass through the spaces between the secondary inductors 41A-41J and will be in intimate thermal contact therewith.

In certain embodiments, the chamber 31 is circular and the secondary inductors 41A-41J are correspondingly formed as flat rings. This shape simplifies obtaining symmetry and uniform heating, but is not necessary to the mode of inductive heating. In other embodiments, other shapes are used. Spacing between adjacent secondary inductors 41A-41J is maintained by spacers 43.

In certain embodiments, where amounts and/or properties of the fluids in the heating system change, it is desirable to modify certain key parameters of the heat exchanger. When a fluid is in contact with a solid surface, heat flux between the fluid and the solid surface, which is the rate of heat energy transfers through the given surface per unit time, can be expressed as follows:

$$q = h \cdot \Delta T$$

wherein $q$ is heat flux (W/m$^2$), $h$ is a heat transfer coefficient (W/m$^2$•K), and $\Delta T$ is the difference in temperature between the solid surface and surrounding fluid area (K). The heat transfer coefficient is a function of the key parameters, for example, conductivity of the solid (e.g., the secondary inductors), gaps between the secondary inductors, etc. Total heat transfer from the solid surface (e.g., secondary conductors) to the fluid can be calculated by multiplying total surface area of the secondary conductors with the heat flux. Therefore, when one changes the fluid to be heated and/or operating conditions, it may be desirable to increase or decrease gaps between the secondary inductors, increase or decrease the number of secondary conductors, change the total surface area of secondary inductors, and/or change materials used in the construction of the secondary inductors. For example, in order to accommodate a higher heating capacity (e.g., higher flow rate), the number and/or the total surface area of the secondary conductors may be increased.

In certain embodiments, the secondary inductors are constructed, for example, of stainless steel. Other conductive materials such as conductive plastics might also be used. In certain embodiments, one or more conductive materials are selected from the group consisting of stainless steel, carbon (graphene), silver, copper, gold, aluminum, tungsten, zinc, nickel, lithium, iron, platinum, tin, carbon steel, lead, titanium, grain oriented electrical steel, manganin, constantan, mercury, nichrome, carbon (graphite) and combinations thereof. In certain embodiments, one chooses less conductive material (e.g., high resistance) to generate increased dissipation heat in the secondary inductors.

In certain embodiments, a conduit/chamber of the present disclosure has at least 1, 2, 4, 6, 8, 10, 12, 14, 16, 18, or 20 secondary inductors.

In certain embodiments, each gap between secondary inductors has a distance of about 0.001" to about 0.1", about 0.001" to about 0.1", about 0.001" to about 0.1", or about 0.02" to about 0.03".

In certain embodiments, total surface area of secondary inductors is about 1 to about 1000 in$^2$, about 1 to about 500 in$^2$, about 1 to about 250 in$^2$, about 10 to about 1000 in$^2$, about 10 to about 500 in$^2$, or about 10 to about 250 in$^2$.

Fluid separator

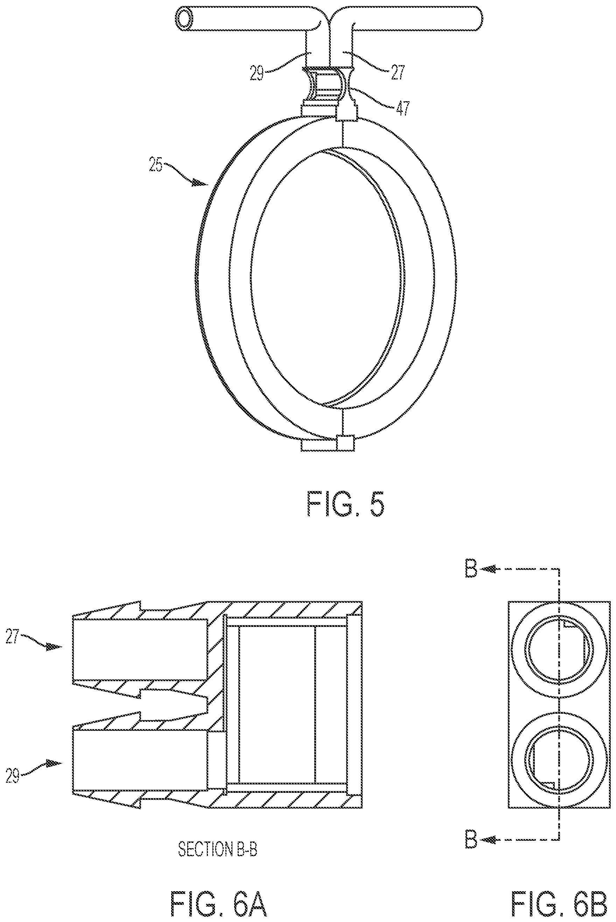

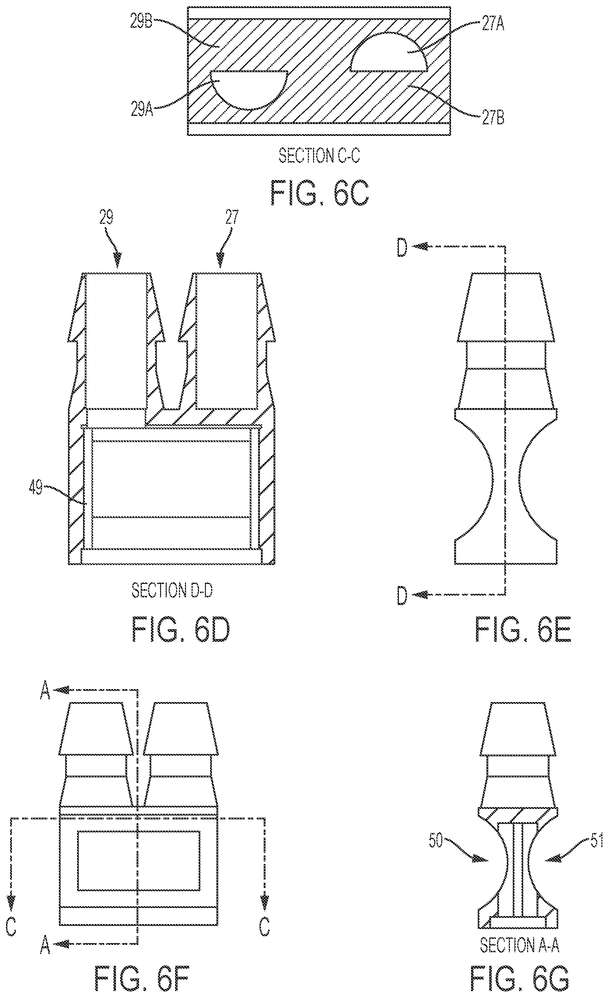

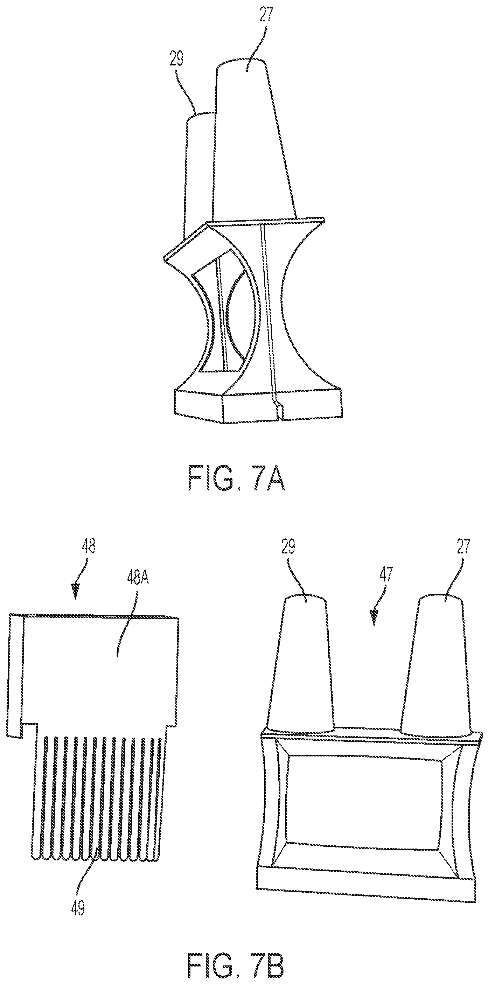

In certain embodiments, a fluid separator is incorporated into a single flow path heating system. The fluid separator may enable a single flow path with minimal modification of the existing system (e.g., same primary inductor, tubing, etc.). An exemplary fluid separator is depicted in FIGS. 6A-6G, 7A, 7B and 8. The fluid separator comprises an inlet nozzle and an outlet nozzle. The inlet nozzle 27 directs unheated (e.g., cold) fluid to the single flow path, while the outlet nozzle 29 receives heated fluid from the single flow path. The inlet and outlet nozzle are substantially parallel to each other to allow flow through the outlet nozzle in an opposite direction to flow through the inlet nozzle.

In certain embodiments, the fluid separator has a housing. The housing contains two sections or chambers, an inlet chamber and an outlet chamber that are separated by a divider. The divider is discussed in more detail below. The inlet fluid (e.g., unheated infusate) first enters through the inlet 27 into the inlet chamber, and then moves into the single fluid path. The fluid while in the single fluid path is heated by the heat exchanger system described previously. The outlet fluid (e.g., heated infusate) exits from the single fluid path, then passes through the outlet chamber and the outlet 29.

In certain embodiments, the inlet 27A and the outlet nozzles 29A are of semi-circular cross-section and are mirror images of each other along the length of the fluid separator (FIG. 6C). This design allows for the physical separation of the inlet fluid and the outlet fluid, and their flow to separate chambers.

Temperatures of the inlet fluid and outlet fluid can be measured at exterior walls of the inlet chamber and the outlet chamber using temperature probes, respectively. A portion of the housing is sufficiently thermally-conductive, so that the temperature of the fluid (e.g., inlet unheated fluid, outlet heated fluid) is substantially identical to the temperature of the housing (e.g., outside of the inlet chamber, outside of the outlet chamber) that is in direct contact with the fluid. In certain embodiments, the housing comprises a notch that can secure a divider. In certain embodiments, the housing has two convex walls (50 and 51) at the inlet chambers and the outlet chambers, from the perspective of each of the inlet chamber and the outlet chamber as shown in FIGS. 6E and 6G. Temperature probes may measure inlet and outlet temperatures at these convex walls.

In certain embodiments, a fluid separator comprises a divider 48. The divider 48 separates the fluid separator housing into two sections or chambers - the inlet chamber and the outlet chamber, separating the unheated fluid in the inlet chamber and the heated fluid in the outlet chamber. The inlet nozzle 27 and the outlet nozzle 29 are connected to different sections or chambers of the fluid separator, so that the divider effectively separates the unheated fluid and the heated fluid.

The divider 48 may have a solid upper portion 48A to satisfactorily separate the inlet chamber from the outlet chamber when the divider is secured in the fluid separator. The divider may also have a lower portion comprising a plurality of elongations 49 to accommodate a plurality of secondary inductors. For example, each elongation of the divider 49 may occupy completely and block each gap between any two successive ring shaped secondary inductors in the plurality of secondary inductors 41A-41J, so that the elongations of the divider prevent mixing of the unheated fluid and the heated fluid that flow between the secondary inductors. In certain embodiments, each thickness of the elongations 49 is substantially identical to each gap between the secondary inductors. In certain embodiments, the width of the notch in the housing of the fluid separator and the thickness of the divider are substantially identical, so that the notch can secure the divider in the fluid separator.

In certain embodiments, the cross section of the inlet nozzle 27A, where it is connected to the inlet chamber is semi-circular as shown in FIG. 6C. Similarly, the cross section of the outlet nozzle 29A, where it is connected to the outlet chamber may be semi-circular. The semi-circular cross-sections of the inlet and the outlet are located on opposite sides of the fluid divider, so that the divider can separate the inlet and the outlet when the divider is positioned therebetween.

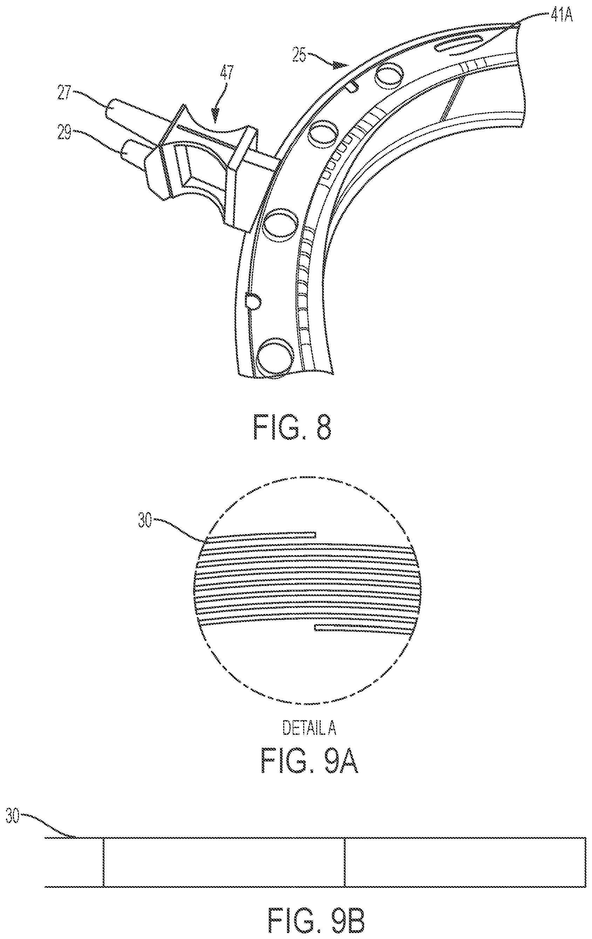

In certain embodiments, the fluid separator is connected to the peripheral area of the conduit, as shown in FIG. 8, so that the inlet nozzle and the outlet nozzle do not disturb alternating magnetic fields created by the primary inductor.

In certain embodiments, seams between the divider and the housing are sealed. In certain embodiments, seams between the plurality of elongations of the divider and the plurality of secondary inductors may be sealed. For example, in certain embodiments adhesive may be applied at seams and cured with ultraviolet light.

In certain embodiments, a fluid separator of the present disclosure is constructed of insulating materials, for example, polymers and/or plastic so that the separator does not disturb the magnetic field in any instance.

Spiral Inductive Tube

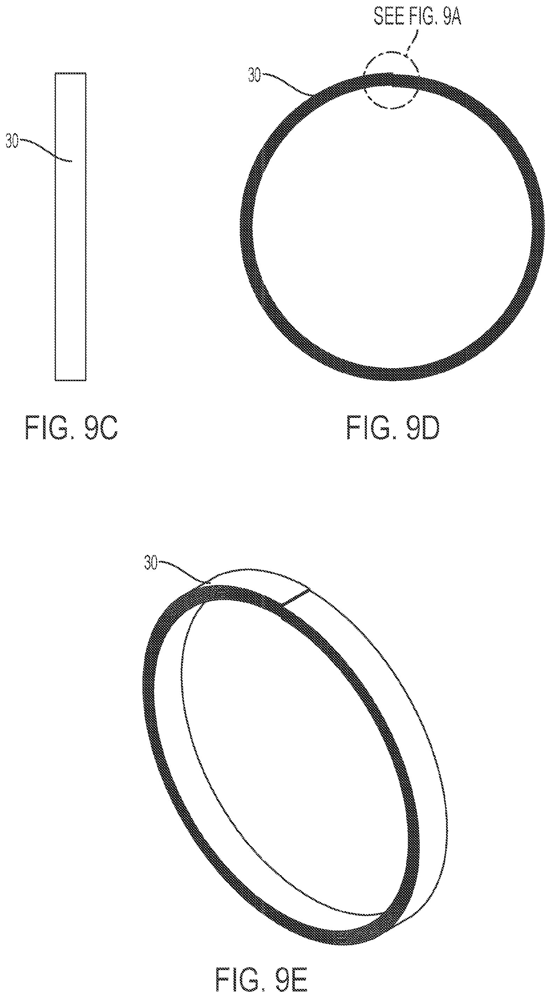

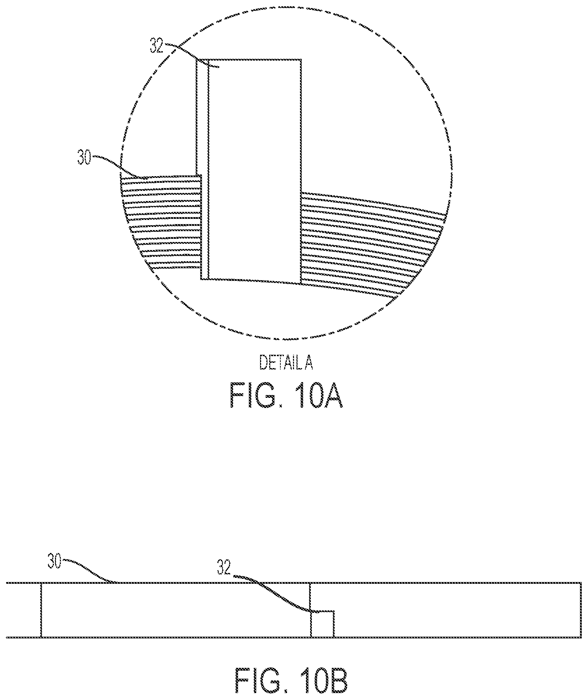

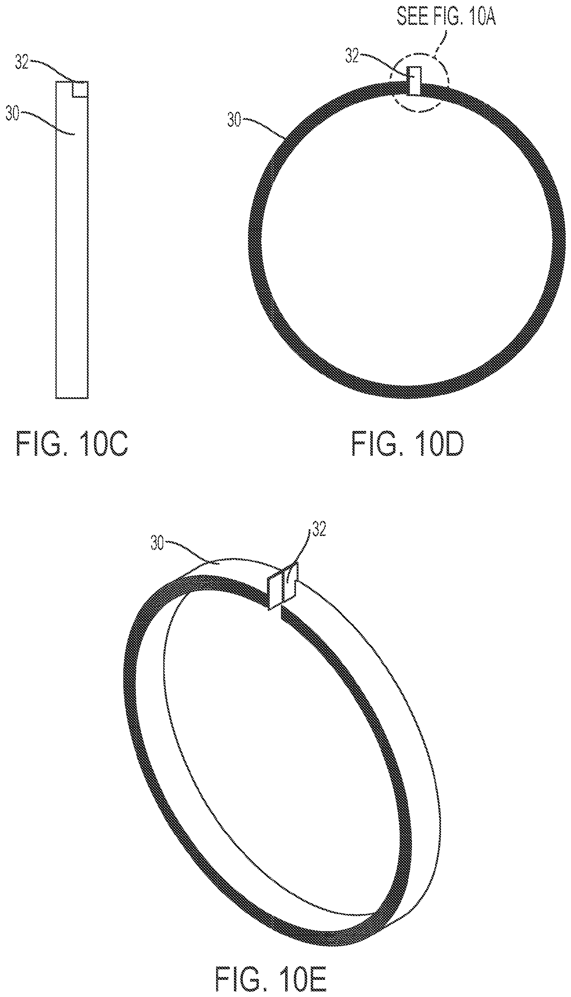

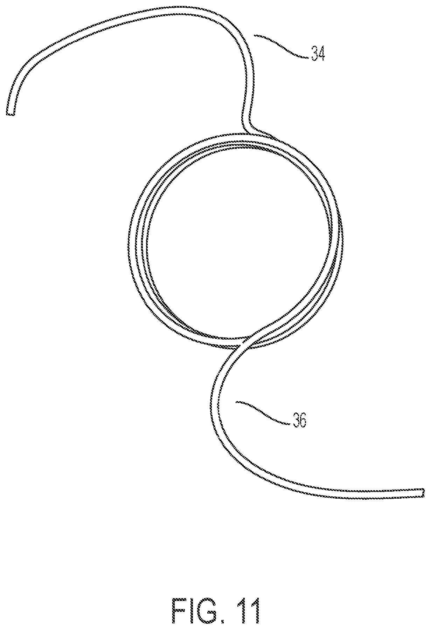

In certain embodiments, a spiral inductive tube provides a single flow path (e.g., instead of a fluid separator, a conduit, and secondary inductors) for the fluid from the inlet of the tube to the outlet. Exemplary spiral inductive tubes 30 are depicted in FIGS. 9A-9E and 10A-10E. A tube (e.g., conductive tube) may be shaped into a circular form. This is repeated multiple times using the same tube, creating the spiral inductive tube 30. The spiral tube 30 comprises one or more circular loops. The spiral tube 30 may comprise one or two extended tails (34 and 36 of FIG. 11), which can be connected to an inlet and/or outlet. The spiral tube may comprise a window 32 as shown in FIG. 10A-10E for measuring temperature of the fluid flowing.

Similar to embodiments with a plurality of thin ring-shaped secondary inductors, a primary inductor comprising a winding 55 wound on a ferrite bobbin core 56 may be inserted into a central opening of the spiral inductive tube. The primary inductor generates magnetic flux passing through the central opening, inductively coupling to the spiral inductive tube and generating local currents therein. An unheated fluid enters from one of the extended tails of the tube (e.g., fluid inlet) and flows within the spiral inductive tube, contacting the inner walls of the spiral inductive tube. Heat generated within the inductive walls of the spiral inductive tube (e.g., due to the alternating magnetic fields from the primary inductor located in the central opening of the spiral inductive tube) is transferred to the fluid flowing in the spiral inductive tube.

In certain embodiments, the spiral inductive tube comprises one or more conductive wires (e.g., copper, stainless steel) that electrically connect loops. The conductive wires further provide electrical shorts within the spiral inductive tube.

In certain embodiments, the spiral inductive tube comprises an insulating housing (e.g., polymer, plastic).

In certain embodiments, where amounts and/or properties of the fluids in the heating system vary, it may be desirable to modify certain key parameters of the spiral inductive tube. For example, it is desirable to increase or decrease inner and/or outer diameters of the spiral inductive tube, increase or decrease the number of windings, change the total surface area of the spiral inductive tube, and/or change materials used in the construction of the spiral inductive tube based on the type and/or volume of fluids to be heated. For example, in order to accommodate a higher heating capacity, the total surface area, and/or windings of the spiral inductive tube may be increased.

In certain embodiments, a spiral inductive tube is constructed of one or more conductive materials selected from the group consisting of stainless steel, carbon (graphene), silver, copper, gold, aluminum, tungsten, zinc, nickel, lithium, iron, platinum, tin, carbon steel, lead, titanium, grain oriented electrical steel, manganin, constantan, mercury, nichrome, carbon (graphite) and combinations thereof.

In certain embodiments, a spiral inductive tube has a total interior surface area of about 1 $in^2$ to about 1000 $in^2$, about 5 $in^2$ to about 1000 $in^2$, about 10 $in^2$ to about 1000 $in^2$, about 1 $in^2$ to about 500 $in^2$, about 1 $in^2$ to about 100 $in^2$, or about 1 $in^2$ to about 50 $in^2$.

In certain embodiments, an inner diameter of a spiral inductive tube ranges from about 1/32" to about 1", from about 1/16" to about 1", from about 1/32" to about 1/2", from about 1/32" to about 1/4", or from about 1/16" to about 1/4".

In certain embodiments, a spiral inductive tube winds about 1 to about 20 times, about 1 to about 10 times, or about 1 to about 5 times.

Operation

As indicated previously, the energy inductively coupled to the secondary inductors 41A-41J or the spiral inductive tube is preferably controlled to maintain a preselected temperature at the outlet of the heater. Circuitry suitable for this purpose is illustrated in FIG. 13. The temperature sensor 51 provides an output signal corresponding to the temperature at the outlet of the heater. This temperature signal is compared with a reference voltage representing a desired temperature, e.g. 42 °C, by an error amplifier designated generally by reference character 63.

The error signal obtained from the error amplifier 63 is applied to a modulator 65 which modulates an amplitude cycle of a low frequency signal obtained from a sine wave oscillator 66. The pulse width of this amplitude modulated signal is in turn modulated, as indicated at 67 by a high frequency signal obtained from an oscillator 71. This results in a signal or waveform having a high frequency carrier, but with an energy content proportional to its low frequency amplitude. This signal is in turn applied through suitable driver circuitry 77 to a bridge type power output circuit 79, which provides alternating current energization of the inductor winding 55.

As will be understood by those skilled in the art, power transferred to the secondary inductors 41A-41J or the spiral inductive tube will be determined essentially by the average power content of the waveform applied to the winding 55, thus, this power will be modulated in accordance with an error signal generated by the error amplifier 63 such that the temperature at the output of a heater is maintained at a value substantially equal to the desired or set point temperature. Further, since the heat is generated in the secondary inductors 41A-41J or the spiral inductive tube themselves which are in intimate thermal contact with the fluid passing through the heater, a very high overall efficiency is obtained. Further, since the volume of fluid within the heater at any given moment is relatively small as compared with other devices, a relatively quick response is obtained and very little fluid is lost or unavailable to a patient, since the volume required to fill the system is correspondingly small.

In certain embodiments, an exemplary system of the present disclosure receives electric power by an Alternating Current (AC) wall outlet. In certain embodiments, the system is operated from battery power.

In certain embodiments, the system offers adjustability of flow rate of a fluid from 1 ml/min to 2000 ml/min, or from 10 ml/min to 2000 ml/min. As discussed herein, the system obviates clogging issues that may result from low flow of improperly treated fluids (e.g., improper or compromised anticoagulants) in dual flow path systems. Thus, the embodiments of the single flow path system described herein provide for advantageous operation at low flow rates, e.g., non-zero flow rates of less than about 100 ml/min, or less than about 50 ml/min, or less than about 40 ml/min, or less than about 30 ml/min, or less than about 20 ml/min, or less than about 15 ml/min, or less than about 10 ml/min.

In certain embodiments, an exemplary system of the present disclosure comprises a bubble trap that separates air from fluid by gravitational force. For example, the bubble trap may have a chamber with an inlet and an outlet. The outlet may be located at the bottom of the chamber. Any air introduced by a pump into a fluid due to gravitational force may separate from the fluid and may move to the top of the chamber, while the heated fluid (e.g., without any air bubbles) exits through the outlet from the bottom of the chamber.

A small part of the system needs to be disposable or replaceable. These parts may be replaced or disposed from use to use. All of the electronics, energizing inductor and magnetic cores can be used repeatedly. In certain embodiments, a conduit or chamber 31 is included as part of a disposable set. In certain embodiments, a plurality of secondary inductors are included as part of a disposable set. In certain embodiments, a fluid separator is included as part of a disposable set. In certain embodiments, a spiral inductive tube is included as part of a disposable set. In certain embodiments, a bubble trap is included as part of a disposable set. In certain embodiments, a housing is included as part of a disposable set. In certain embodiments, a disposable set is for a single use. A disposable set is constructed of materials that can be sterilized and made pyrogen free by conventional methods and so that single uses thereof are economically feasible.

In certain embodiments, components that the biological fluid (e.g., blood) contacts are biocompatible and/or sterilizable or replaceable.

Slack time heating system

Administration of cold blood and other fluids at high flow rates (e.g., greater than 500 ml/min, or greater than 750 ml/min) using an in line warming system requires the delivery of high quantities of power to the fluids. To infuse cold fluids at a rate in excess of these levels would require more energy than the typical AC outlet can supply. Certain systems store thermal energy in a separate fluid (e.g., water or oil, not infusate) for transferring the stored heat from the fluid to the infusate during times of need. Drawbacks of this approach include the necessity to later transfer energy to the infusate before/during infusion with the limited heat transfer rate of the system (e.g., due to a limited surface area between water/oil and infusate) and the potential contamination of the infusate by the fluid bath.

The present disclosure is directed to a slack-time heating system that utilizes excess heating capacity of fluid heaters to pre-warm fluid in a reservoir. The present disclosure describes a heating system to store heat energy within a reservoir (e.g., in infusate) during off peak periods (e.g., when low flow is required) for later use (e.g., when high flow is required).

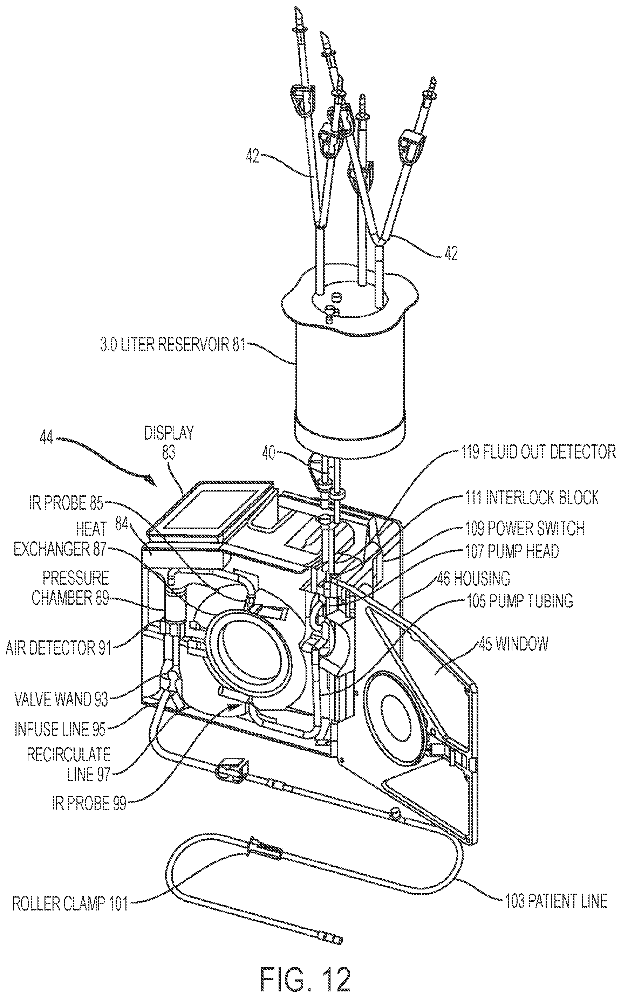

FIG. 12 depicts an exemplary slack-time heating system. The system comprises a large volume reservoir 81 connected to a pump tubing 105 threaded through a roller pump head further connected to a heat exchanger 87. The fluid path continues to a pressure chamber 89 and then directs one path to a patient via a patient line 103 or back to the reservoir 81 via a recirculation line 97. Therapeutic infusion fluid is provided to the heating system from either one or more conventionally available IV type bags (not shown). Fluid tubular lines (not shown) and "Y" connectors 42, direct infusion fluid to reservoir 81, for storage and further use as will be described in greater detail hereinafter. Additional tubular fluid lines 40 direct the fluid from reservoir 81 to and through a therapeutic fluid processing device 44. Therapeutic fluid after being processed by device 44 is directed into a patient tubular fluid feed line 103.

Therapeutic fluid processing device 44 is of a weight, size configuration that permit it to be mounted to, and utilized when, carried by a conventional IV pole (not shown) whether, or not, such IV pole is provided with a wheel base (not shown). Device 44 may just as easily be utilized when positioned on a substantially horizontal surface such as a table, cabinet or the like as long as it is in proximity to the patient that is to undergo the intended procedure.

An air detector 91 is connected by a suitable vacuum line (not shown) to the vacuum regulator/ wall suction (not shown) of the location (hospital, etc.) where patient is to be treated. Device 44 includes a housing 46 (FIGS. 2-5); which includes a top, a bottom, a left side, a right side and a back which together form a housing body and define there within a component space that houses the heating system. An access door, hingedly connected to housing body permits access into component space and to mechanisms and components housed and mounted within housing 46; as will be explained in greater detail herein after. Windows 45 formed through the access door, are covered with "Plexiglas", or similar transparent material, to facilitate observance of the processing taking place by device 44.

A conventionally available magnetic induction heater or heat exchanger 87 is securely positioned within component space of housing 46. Suitable and conventional electric power is provided to therapeutic fluid processing device 44, through a power cord (not shown) that is to be connected to an electric outlet (not shown), and there from to heat exchanger 87. A substantially conventional computer 84, also fixedly positioned within housing 46, along with its control and operating software and/or hardware, also receives power through the power cord (not shown). A touch screen display 83 connected to the computer 84 projects outside the housing 46. Device 44 may also be provided with back-up batteries (not shown) (rechargeable or otherwise) also housed within housing 46 and suitably connected to the components and mechanisms there within that require electric power to operate. A conventional roller-type peristaltic pump, also positioned within housing body 46 also receives suitable electric power through the power cord (not shown) and operates under control of computer 84 and its associated software / hardware.

Positioning grooves are formed in housing 46, to receive specific sections of tubular fluid lines. A temperature IR probe 85 is mounted proximate a fluid entry to heat exchanger 87 and another temperature probe 99 at a fluid exit from heat exchanger 87. Temperature probes 85, 99 are suitably connected to power and computer 84, and its software/hardware, and they are of the infrared type but other types of temperature probes may just as well be so mounted and used. A substantially semi-cylindrical pressure chamber well 80, formed in housing 46, uses a pressure transducer (not shown), which is also mounted in housing 46 proximate to an air detector 91. A valve wand 93 is also mounted in housing 46 between fluid line positioning grooves. A fluid out detector (not shown) is mounted proximate to fluid entry into device 44 and its fluid pump. The fluid out line can be selectively connected to the infuse line 95 that connects to the patient line 103 (e.g., to send infusate / fluid to the patient) or recirculate line 97 to send infusate / fluid to the reservoir 81 for storage.

In certain embodiments, a slack-time heating system comprises a diversion valve 93 that controls the ratio between a flow in a patient line and a flow in a recirculation line. When the diversion valve is in a recirculation position (e.g., the patient line is obstructed, while the recirculation line is opened), the pump 107 will cause fluid in a bubble trap to flow back to the reservoir (e.g., exit from the top).

In certain embodiments, the slack-time heating system described herein utilizes the heating system when it is not being actively used to infuse a patient. Then the fluid is sent from the reservoir to the heat exchanger (e.g., induction heater), is warmed, and is resent to the reservoir. Then, when the fluid (e.g., blood) is sent to the patient, pre-warmed fluid from the reservoir again passes through the heat exchanger before being sent to the patient. The amount of thermal energy that needs to be transferred to the fluid (e.g., infusate) is smaller, if the temperature difference the heat exchanger needs to achieve to heat the fluid (e.g., infusate) is lower (e.g., if the fluid going into the heat exchanger from the reservoir is pre-warmed). Thus, during a medical procedure, when the heating system is not needed, fluid can pass through the heat exchanger, and then reside in the reservoir until needed, thereby storing thermal energy. Thus, the system can heat the fluid rapidly when the infusion is needed. As the system does not use other fluids to heat the infusate, the system also does not suffer from a risk of contamination, unlike water-based heat exchangers.

In certain embodiments, reservoirs of the present disclosure mix unheated fluid and heated fluid.

In certain embodiments, the ratio of a flow in a patient line to a flow in a recirculation line is between 100:1 and 1:100.

In certain embodiments, the slack time heating system utilizes a single flow path as described in the present disclosure.

Vacuum release valve

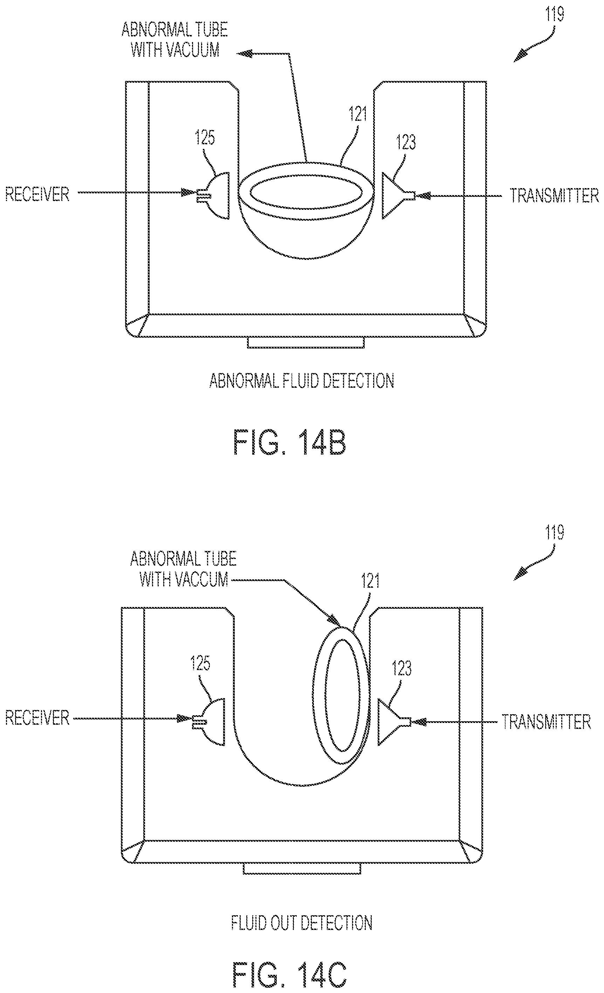

The fluid heating system has a sensor (e.g., fluid out sensor) 119 connected to an inflow tubing 121 (e.g., from an infusate reservoir to fluid heaters) to detect if an infusate reservoir is empty. The sensor comprises a transmitter 123 and a receiver 125, and measures velocity of ultrasound waves from the transmitter 123 to the receiver 125, (e.g., ultrasound travels faster through fluids than air) as shown in FIG. 14A to distinguish between a fluid-filled inflow tubing and tubing when fluid level in the infusate reservoir is low or zero. The inflow tubing 121 is located between the transmitter 123 and the receiver 125. When the fluid level in the infusate reservoir is low (or zero), the pressure in the infusate reservoir and the inflow tubing is lower than the atmospheric pressure. Therefore, the non-rigid inflow tubing 121 deforms to minimize the pressure difference. For example, the cross-section area of the inlet tubing reduces, or the cross-section of the inflow tubing becomes elliptic, as shown in FIGS. 14B and 14C. The sensor measures this deformation of the inflow tubing to detect fluid availability in the tubing and therefore, the reservoir. Since ultrasound waves travel faster through fluids than air, a fluid-filled tubing results in higher velocity ultrasound waves, than a collapsed tubing that causes air to fill any additional space between the transmitter and receiver of the sensor.

The direction of deformation affects detectability of fluid levels in the inflow tubing by the sensor. The pressure difference between the inside of the non-rigid inflow tubing and the outside atmospheric pressure may result in the tubing collapsing from a circular cross-section into an elliptical cross-section. For example, a major axis of the elliptic cross-section of the collapsed inflow tubing can be perpendicular to a line between the transmitter and the receiver as shown in FIG. 14C, or parallel to that line as shown in FIG. 14B. If the major axis of the elliptic cross-section of the collapsed inflow tubing is not parallel (e.g., perpendicular) to the line between the transmitter and the receiver, the sensor can measure the deformation properly as the ultrasound now travels in air more than before the deformation of the inflow tubing occurred. However, if the major axis of the elliptic cross-section of the collapsed inflow tubing is parallel to a line between the transmitter and the receiver, the sensor cannot detect the deformation because of lack of air in the ultrasound beam path.

The present disclosure is directed to a fluid heating system that includes a vacuum release valve to prevent the undesired orientation of the deformed inflow tubing. In certain embodiments, the vacuum release valve supplies air to the tubing to reduce pressure difference between the inflow tubing and the surroundings, preventing the deformation of the inflow tubing.

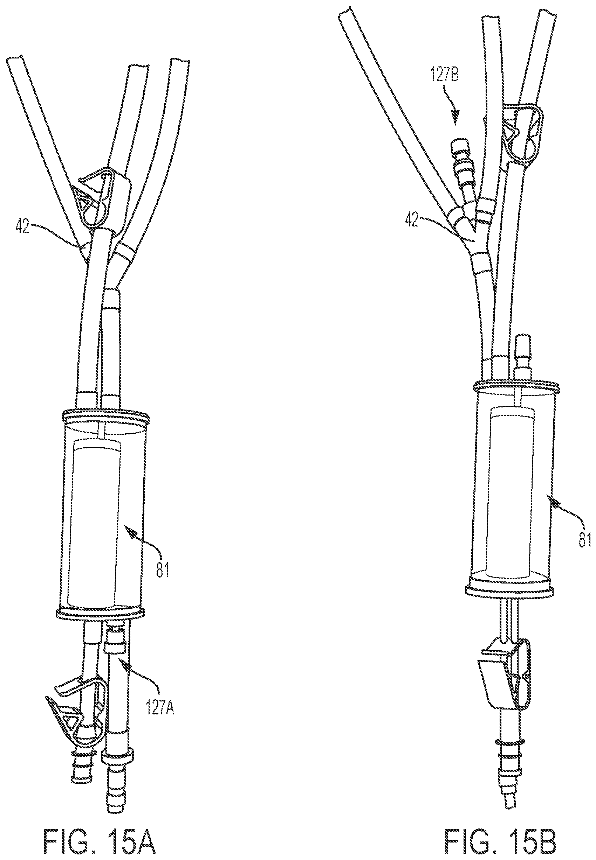

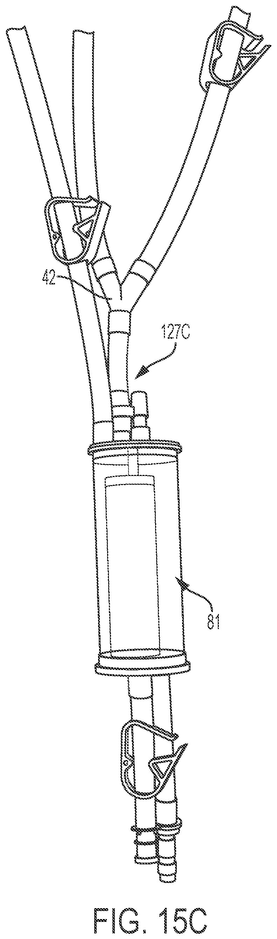

FIGS. 15A-15C depict exemplary disposable sets with vacuum release valves. In certain embodiments, the vacuum release valve 127A is connected on the bottom of the reservoir (FIG. 15A). The inflow tubing and the vacuum release valve may be parallel to each other. In certain embodiments, the vacuum release valve 127C is connected on the top of the reservoir (FIG. 15C). In certain embodiments, the vacuum release valve is indirectly connected to the reservoir. The reservoir may comprise a tubing attached to the top of the reservoir (FIG. 15B). The vacuum release valve 127B may be connected to the reservoir through the tubing.

Any type of suitable vacuum release valve may be used in accordance with the disclosure. In certain embodiments, the vacuum release valve may comprise a housing and a regulator. In certain embodiments, the vacuum release valve may operate automatically (e.g., without an operator). The vacuum release valve is normally closed (e.g., the vacuum release valve does not allow air into the inflow tubing, e.g., the regulator presses against a portion of the housing, thereby blocking air passage). When the pressure difference between the system (e.g., the reservoir, inflow tubing) and the atmosphere reaches a pre-determined value (e.g., 0.5, 1, 2, 3, 4, 5, 6, 7, 8, 9, or 10 psi), the vacuum release valve opens (e.g., the valve allows air to flow into the system). The pre-determined value may be manipulated by varying an external force applied to the regulator. For example, if the pressure difference between the system (e.g., the reservoir, inflow tubing) and the atmosphere exceeds the applied force per area, the valve opens. The force may be applied mechanically (e.g., spring, diaphragm) to the regulator.

In certain embodiments, the vacuum release valve supplies air to the disposable set when the pressure of the disposable set is 0.5, 1, 2, 3, 4, 5, 6, 7, 8, 9, or 10 psi lower than atmospheric pressure.

In certain embodiments, the vacuum release valve comprises a filter, and/or a sanitation unit to provide sterile air to the system.

In certain embodiments, a vacuum release valve is included as part of a disposable set.

EXEMPLIFICATION

Single Flow Path

The present example describes, among other things, an exemplary operation of single flow path induction heater with an exemplary spiral inductive tube.

As shown in FIG. 11, aluminum tubing with an inner diameter of 1/8" and an outer diameter of 3/16" was shaped into a circular form to be operated within the Belmont® Rapid Infuser by Belmont. Circular loops with 2.5 overlapping turns and extended tails were created. Copper wire was wrapped around the input and output in order to provide electrical connection with in the tubing. The tails are positioned in a fashion so that the input and output thermal detectors are as close as possible to the input and the output. The spiral inductive tube was successfully installed in the Belmont® Rapid Infuser. The system pumped fluid through the tubing. Temperature of the outlet fluid was increased relative to temperature of the inlet fluid.

Vacuum Release Valve

The present example describes, among other things, exemplary operations of a rapid heating system with exemplary vacuum release valves.

Disposable sets (e.g., The Belmont® 3-Spike Disposable Set) with various vacuum release valves as shown in FIGS. 15A-15C were tested with the Belmont Rapid Infuser. A vacuum release valve was connected to the 4.4 L reservoir (FIG. 15A), a y-connection at the intersection of tubing (FIG. 15B), or onto the filter assembly (FIG. 15C). The modified disposable sets and an unmodified set were then tested. Testing mediums were water, sodium chloride and water at 35.7 grams per 100 mL, and a 50 / 50 mix of water and glycerin by volume. Each modified disposable set was tested at a wide range of flow rates. While the unmodified set was clamped off due to deformation of the inflow tubing as pressure decreased, the vacuum release valve of the modified sets allowed air to be drawn into the set, and did not experience clamping off. Once the reservoir was sufficiently emptied, air would be drawn into the fluid path causing the fluid out detector to register the low levels of fluid in the heating system and to turn off the machine properly. The valve worked successfully with all testing mediums and at any flow rate. The addition of the vacuum release valve prevented the improper deformation of tubing.

**Claims**

1. A system for heating a fluid, the system comprising:

    a conduit (25) defining a central opening;
    a primary inductor at least partially within the central opening;
    one or more secondary inductors (41A-41J) within the conduit; and
    non-rigid inflow tubing (121) connected to the conduit;

    wherein each of the one or more secondary inductors is substantially parallel, the one or more secondary inductors being separated by gaps or spaces,
    wherein the primary inductor comprises a coil or a winding (55),
    wherein, when the primary inductor is energized, the coil generates magnetic flux passing through the central opening,
    **characterised in that** the system further comprises:

    a reservoir (81) for containing an infusate;
    a vacuum release valve (127A, 127B) connected to the reservoir; and
    a sensor (119) to detect fluid availability in the inflow tubing, comprising a transmitter (123) and a receiver (125), the sensor configured to measure a velocity of ultrasound waves from the transmitter (123) to the receiver (125), the inflow tubing (121) being disposed between the transmitter (123) and the receiver (125),
    wherein, when pressure in the inflow tubing (121) is lower than atmospheric pressure, the vacuum release valve (127A, 127B) allows flow of air into the inflow tubing (121), thereby preventing deformation of the inflow tubing (121).

2. The system according to claim 1, wherein the vacuum release valve (127A, 127B) allows flow of air into the inflow tubing when pressure in the inflow tubing (121) is 2 psi lower than atmospheric pressure.

3. The system according to claim 1 or claim 2, wherein the conduit (25) provides a single fluid flow path.

4. The system according to claim 3, wherein the single fluid flow path has a conformation such that fluid is not in contact with the primary inductor.

5. The system according to any one of the preceding claims, further comprising a fluid separator (47) comprising an inlet nozzle (27) and an outlet nozzle (29), wherein the inlet nozzle (27) directs an inlet flow to the conduit through the fluid separator (47) and the outlet nozzle (29) receives an outlet flow from the conduit through the fluid separator (47).

6. The system according to any one of the preceding claims, wherein the conduit (25) comprises a plurality of spacers (43) which provides the gaps between the one or more secondary inductors (41A-41J).

7. The system according to any one of the preceding claims, wherein the one or more secondary inductors (41A-41J) transfers heat to fluid within the conduit (25).

8. The system according to any one of the preceding claims, wherein the vacuum release valve (127A, 127B) is connected to the top of the reservoir (81), or the bottom of the reservoir (81).

9. The system according to any one of the preceding claims, wherein the reservoir (81) further comprises a tubing attached to the top of the reservoir, and the vacuum release valve (127B) is connected to the reservoir (81) through the tubing.

10. The system according to any one of the preceding claims, wherein the vacuum release valve (127A, 127B) comprises an air filter.

11. The system according to any one of the preceding claims, further comprising a bubble trap for removing air bubbles from fluid flowing through the system.

12. The system according to any one of the preceding claims, wherein the conduit (25) is toroid-shaped.

13. The system according to any one of the preceding claims, where each of the secondary inductors (41A-41J) is ring-shaped and flat.

**Patentansprüche**

1. System zum Erwärmen einer Flüssigkeit, wobei das System Folgendes umfasst:

eine Leitung (25), die eine zentrale Öffnung definiert;
einen Primärinduktor mindestens teilweise innerhalb der zentralen Öffnung;
einen oder mehrere Sekundärinduktoren (41A-41J) innerhalb der Leitung; und
einen nicht starren Einströmschlauch (121), der mit der Leitung verbunden ist;
wobei jeder von dem einen oder den mehreren Sekundärinduktoren im Wesentlichen parallel ist, wobei der eine oder die mehreren Sekundärinduktoren durch Lücken oder Zwischenräume voneinander getrennt sind,
wobei der Primärinduktor eine Spule oder eine Wicklung (55) umfasst,
wobei, wenn der Primärinduktor erregt ist, die Spule einen magnetischen Fluss erzeugt, der durch die zentrale Öffnung verläuft,
**dadurch gekennzeichnet, dass** das System ferner Folgendes umfasst:

ein Reservoir (81) zum Enthalten eines Infusats;
ein Vakuumentlastungsventil (127A, 127B), das mit dem Reservoir verbunden ist; und
einen Sensor (119) zum Erfassen der Flüssigkeitsverfügbarkeit in dem Einströmschlauch, umfassend einen Sender (123) und einen Empfänger (125), wobei der Sensor konfiguriert ist, um eine Geschwindigkeit von Ultraschallwellen von dem Sender (123) zu dem Empfänger (125) zu messen, wobei der Einströmschlauch (121) zwischen dem Sender (123) und dem Empfänger (125) angeordnet ist,
wobei, wenn der Druck in dem Einströmschlauch (121) niedriger als der Atmosphärendruck ist, das Vakuumentlastungsventil (127A, 127B) einen Luftstrom in den Einströmschlauch (121) zulässt, wodurch eine Verformung des Einströmschlauchs (121) verhindert wird.

2. System nach Anspruch 1, wobei das Vakuumentlastungsventil (127A, 127B) einen Luftstrom in den Einströmschlauch zulässt, wenn der Druck in dem Einströmschlauch (121) 2 psi niedriger als der Atmosphärendruck ist.

3. System nach Anspruch 1 oder Anspruch 2, wobei die Leitung (25) einen einzelnen Flüssigkeitsströmungsweg bereitstellt.

4. System nach Anspruch 3, wobei der einzelne Flüssigkeitsströmungsweg eine derartige Konformation aufweist, dass die Flüssigkeit nicht mit dem Primärinduktor in Kontakt kommt.

5. System nach einem der vorhergehenden Ansprüche, ferner umfassend einen Flüssigkeitsabscheider (47), umfassend eine Einlassdüse (27) und eine Auslassdüse (29), wobei die Einlassdüse (27) einen Einlassstrom zu der Leitung durch den Flüssigkeitsabscheider (47) leitet und die Auslassdüse (29) einen Auslassstrom von der Leitung durch den Flüssigkeitsabscheider (47) aufnimmt.

6. System nach einem der vorhergehenden Ansprüche, wobei die Leitung (25) eine Vielzahl von Abstandshaltern (43) umfasst, die die Lücken zwischen dem einen oder den mehreren Sekundärinduktoren (41A-41J) bereitstellt.

7. System nach einem der vorhergehenden Ansprüche, wobei der eine oder die mehreren Sekundärinduktoren (41A-41J) Wärme an Flüssigkeit innerhalb der Leitung (25) übertragen.

**8.** System nach einem der vorhergehenden Ansprüche, wobei das Vakuumentlastungsventil (127A, 127B) mit der Oberseite des Reservoirs (81) oder der Unterseite des Reservoirs (81) verbunden ist.

**9.** System nach einem der vorhergehenden Ansprüche, wobei das Reservoir (81) ferner einen Schlauch umfasst, der an der Oberseite des Reservoirs angebracht ist, und das Vakuumentlastungsventil (127B) durch den Schlauch mit dem Reservoir (81) verbunden ist.

**10.** System nach einem der vorhergehenden Ansprüche, wobei das Vakuumentlastungsventil (127A, 127B) einen Luftfilter umfasst.

**11.** System nach einem der vorhergehenden Ansprüche, ferner umfassend eine Blasenfalle zum Entfernen von Luftblasen aus Flüssigkeit, das durch das System strömt.

**12.** System nach einem der vorhergehenden Ansprüche, wobei die Leitung (25) toroidförmig ist.

**13.** System nach einem der vorhergehenden Ansprüche, wobei jeder der Sekundärinduktoren (41A-41J) ringförmig und flach ist.


**Revendications**

**1.** Système destiné à chauffer un fluide, ledit système comprenant :

un conduit (25) définissant une ouverture centrale ;
un inducteur primaire situé au moins partiellement à l'intérieur de l'ouverture centrale ;
un ou plusieurs inducteurs secondaires (41A-41J) situés à l'intérieur du conduit ; et
un tube d'admission non rigide (121) relié au conduit ;
dans lequel chacun du ou des inducteurs secondaires est sensiblement parallèle, le ou les inducteurs secondaires étant séparés par des interstices ou des espaces,
dans lequel l'inducteur primaire comprend une bobine ou un enroulement (55),
dans lequel, lorsque l'inducteur primaire est excité, la bobine génère un flux magnétique traversant l'ouverture centrale,
**caractérisé en ce que** le système comprend en outre :

un réservoir (81) destiné à contenir un perfusat ;
une soupape casse-vide (127A, 127B) reliée au réservoir ; et

un capteur (119) destiné à détecter une présence de fluide dans le tube d'admission, comprenant un émetteur (123) et un récepteur (125), le capteur étant configuré pour mesurer la vitesse des ondes ultrasonores de l'émetteur (123) vers le récepteur (125), le tube d'admission (121) étant disposé entre l'émetteur (123) et le récepteur (125),
dans lequel, lorsque la pression dans le tube d'admission (121) est inférieure à la pression atmosphérique, la soupape casse-vide (127A, 127B) permet un écoulement d'air dans le tube d'admission (121), empêchant ainsi la déformation du tube d'admission (121).

**2.** Système selon la revendication 1, dans lequel la soupape casse-vide (127A, 127B) permet un écoulement d'air dans le tube d'admission lorsque la pression dans le tube d'admission (121) est inférieure de 2 psi à la pression atmosphérique.

**3.** Système selon la revendication 1 ou 2, dans lequel le conduit (25) fournit un trajet d'écoulement de fluide unique.

**4.** Système selon la revendication 3, dans lequel le trajet d'écoulement de fluide unique a une conformation telle que le fluide n'est pas en contact avec l'inducteur primaire.

**5.** Système selon l'une quelconque des revendications précédentes, comprenant en outre un séparateur de fluide (47) comprenant une buse d'entrée (27) et une buse de sortie (29), dans laquelle la buse d'entrée (27) dirige un écoulement d'entrée vers le conduit à travers le séparateur de fluide (47) et la buse de sortie (29) reçoit un écoulement de sortie provenant du conduit à travers le séparateur de fluide (47).

**6.** Système selon l'une quelconque des revendications précédentes, dans lequel le conduit (25) comprend une pluralité d'entretoises (43) qui créent des interstices entre le ou les inducteurs secondaires (41A-41J).

**7.** Système selon l'une quelconque des revendications précédentes, dans lequel le ou les inducteurs secondaires (41A-41J) transfèrent de la chaleur au fluide à l'intérieur du conduit (25).

**8.** Système selon l'une quelconque des revendications précédentes, dans lequel la soupape casse-vide (127A, 127B) est reliée au sommet du réservoir (81) ou au fond du réservoir (81).

**9.** Système selon l'une quelconque des revendications précédentes, dans lequel le réservoir (81) comprend en outre un tube fixé au sommet du réservoir, et la soupape casse-vide (127B) est reliée au réservoir (81) par l'intermédiaire du tube.

10. Système selon l'une quelconque des revendications précédentes, dans lequel la soupape casse-vide (127A, 127B) comprend un filtre à air.

11. Système selon l'une quelconque des revendications précédentes, comprenant en outre un piège à bulles destiné à éliminer les bulles d'air du fluide s'écoulant à travers le système.

12. Système selon l'une quelconque des revendications précédentes, dans lequel le conduit (25) est de forme toroïdale.

13. Système selon l'une quelconque des revendications précédentes, dans lequel chacun des inducteurs secondaires (41A-41J) est de forme annulaire et plat.

FIG. 1

FIG. 2

FIG. 3

FIG. 4

FIG. 5

SECTION B-B

FIG. 6A

FIG. 6B

SECTION C-C

FIG. 6C

SECTION D-D

FIG. 6D

FIG. 6E

FIG. 6F

SECTION A-A

FIG. 6G

27

29

FIG. 7A

48

48A

29

47

27

49

FIG. 7B

FIG. 8

DETAIL A

FIG. 9A

FIG. 9B

SEE FIG. 9A

30

FIG. 9C

FIG. 9D

30

30

FIG. 9E

DETAIL A

FIG. 10A

FIG. 10B

FIG. 10C

FIG. 10D

FIG. 10E

FIG. 11

3.0 LITER RESERVOIR 81

42

42

44

DISPLAY 83

119 FLUID OUT DETECTOR

111 INTERLOCK BLOCK

109 POWER SWITCH

107 PUMP HEAD

46 HOUSING

105 PUMP TUBING

45 WINDOW

IR PROBE 85

HEAT 84

EXCHANGER 87

PRESSURE CHAMBER 89

AIR DETECTOR 91

VALVE WAND 93

INFUSE LINE 95

RECIRCULATE LINE 97

IR PROBE 99

ROLLER CLAMP 101

103 PATIENT LINE

40

FIG. 12

28

66 65 71 H.F. OSC. 67 79 55

SINE WAVE OSC. → AMPLITUDE MODULATOR → PULSE WIDTH MODULATOR → POWER DRIVER

77

63 $V_{REF}$

51

FIG. 13

NORMAL TUBE WITH FLOW

121

125

RECEIVER

123

TRANSMITTER

119

NORMAL FLUID DETECTION

FIG. 14A

ABNORMAL FLUID DETECTION

FIG. 14B

FLUID OUT DETECTION

FIG. 14C

42

81

127A

**FIG. 15A**

127B

42

81

**FIG. 15B**

FIG. 15C

130

132
Heat inlet infusate via fluid heater

134
Direct outlet infusate from heater into
either a patient line or a recirculation line

136
Provide outlet infusate to patient though
patient line

138
Direct outlet infusate to reservoir though
recirculation line to provide heat to an
infusate contained in the reservoir

FIG. 16

EP 3 548 124 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5319170 A
- US 7819835 B
- US 9737672 B
- US 2009012655 A1
- US 6280422 B1
- US 2001025160 A1
- US 6175688 B
- US 6236809 B
- US 6480257 B